# FASCICULE DE BREVET EUROPEEN

(11) **EP 2 214 632 B1**
(45) Date de publication et mention de la délivrance du brevet: **07.03.2012**
(21) Numéro de dépôt: 08844247.0
(22) Date de dépôt: 31.10.2008
(51) Int. Cl.: A61K 8/58, A61Q 5/08, A61Q 5/10

(54) **ECLAIRCISSEMENT ET/OU COLORATION DE FIBRES KERATINIQUES HUMAINES AU MOYEN D'UNE COMPOSITION COMPRENANT UN COMPOSE AMINOSILICIE PARTICULIER**
AUFHELLUNG UND/ODER FÄRBUNG MENSCHLICHER KERATINFASERN MITTELS EINER ZUSAMMENSETZUNG MIT EINER BESTIMMTEN AMINOSILICIUMVERBINDUNG
LIGHTENING AND/OR DYEING OF HUMAN KERATIN FIBRES BY MEANS OF A COMPOSITION COMPRISING A PARTICULAR AMINO SILICON COMPOUND

(30) Priorité: 31.10.2007 FR 0758744
(43) Date de publication de la demande: 11.08.2010
(73) Titulaire: L'Oréal, 75008 Paris (FR)
(72) Inventeur: SAMAIN, Henri, F-91570 Bievres (FR); HERCOUET, Leïla, F-93360 Neuilly Plaisance (FR)
(74) Mandataire: Wattremez, Catherine
(86) Numéro de dépôt international: PCT/FR2008/051975
(87) Numéro de publication internationale: WO 2009/056778

(56) Documents cités:
- EP-A- 1 298 135
- US-A1- 2006 110 351

## Description

La présente invention a pour objet un procédé d'éclaircissement et/ou de coloration de fibres kératiniques humaines, mettant en oeuvre d'une part une composition comprenant un composé aminosilicié particulier d'autre part une composition oxydante.

Elle concerne également une composition particulière comprenant un composé aminosilicié particulier et au moins un corps gras.

Elle concerne enfin un dispositif à plusieurs compartiments dont l'un au moins comprend la composition précitée et au moins un autre comprenant une composition oxydante.

Parmi les méthodes de coloration des fibres kératiniques humaines, telles que les cheveux, on peut citer la coloration d'oxydation ou permanente. Plus particulièrement, ce mode de coloration met en oeuvre un ou plusieurs précurseurs de colorant d'oxydation, plus particulièrement une ou plusieurs bases d'oxydation éventuellement associées à un ou plusieurs coupleurs.

Habituellement, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées, par un processus de condensation oxydative.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

Le procédé de coloration consiste à mettre en contact le ou les précurseurs de colorants d'oxydation avec un agent oxydant, qui est de préférence le peroxyde d'hydrogène, dans des conditions alcalines. L'une des difficultés réside dans le fait que l'agent alcalin le plus couramment utilisé est l'ammoniaque, dont le rôle est d'ajuster le pH de la composition à un pH alcalin pour permettre la dégradation de l'agent oxydant. Ainsi l'oxygène formé provoque la condensation des précurseurs de colorants d'oxydation ainsi qu'un éclaircissement de la fibre dû à la dégradation de la mélanine présente. L'agent alcalinisant a également un autre rôle, celui de faire gonfler la fibre kératinique afin de favoriser la pénétration de l'oxydant ainsi que des colorants à l'intérieur de la fibre.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

Un autre mode de coloration employé est celui de la coloration directe ou semi-permanente. Ce procédé consiste à appliquer sur les fibres kératiniques des colorants directs qui sont des molécules colorées et colorantes, ayant une affinité pour les fibres, à laisser poser pour permettre aux molécules de pénétrer, par diffusion, à l'intérieur de la fibre, puis à les rincer.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques.

Ce mode de coloration ne nécessite pas l'emploi d'un agent oxydant à moins que l'on ne souhaite obtenir simultanément à la coloration, un éclaircissement de la fibre. Dans ce dernier cas, on procède comme pour la coloration d'oxydation, c'est-à-dire en mettant en contact les fibres kératiniques avec la composition tinctoriale en présence d'un agent oxydant, plus particulièrement le peroxyde d'hydrogène, en condition alcaline généralement en présence d'ammoniaque. On se retrouve donc également confronté aux mêmes difficultés que celles détaillées auparavant pour la coloration d'oxydation.

Outre les procédés de coloration, il est également courant de mettre en oeuvre des procédés d'éclaircissement dans lesquels on met en contact les fibres kératiniques avec une composition oxydante en conditions alcalines. Ces procédés consistent à uniquement dégrader la mélanine du cheveu, dans une plus ou moins forte mesure en fonction de l'agent oxydant choisi. Ainsi un sel peroxygéné conduit en général à un éclaircissement plus prononcé qu'en employant du peroxyde d'hydrogène seul en conditions alcalines. Cependant, quelque soit l'agent oxydant retenu, les procédés d'éclaircissement nécessitent l'emploi de peroxyde hydrogène en conditions alcalines et en particulier en présence d'ammoniaque pour former ou accélérer la formation d'oxygène. Par conséquent, on rencontre là encore, les mêmes difficultés que celles des procédés de coloration mis en oeuvre en présence d'un agent oxydant et d'ammoniaque.

L'un des objectifs de la présente invention est donc de proposer des compositions de coloration et/ou d'éclaircissement des fibres kératiniques humaines destinées à être mises en oeuvre en présence d'un agent oxydant qui ne présentent pas les inconvénients que les compositions existantes, dus à la présence de teneurs importantes en ammoniaque, tout en restant au moins aussi efficaces, tant sur le plan de l'éclaircissement que sur celui de la coloration, avec en particulier de bonnes performances en terme de chromaticité, de puissance et d'homogénéité.

Il est à noter qu'il était loin d'être évident d'employer comme agents alcalinisants, des composés aminosiliciés correspondants à ceux de la formule (I) dans ce type de procédés.

En effet, en présence de quantités d'eau suffisantes, la plupart de ces composés aminosiliciés s'hydrolysent et se condensent rapidement. On aurait donc pu s'attendre à une baisse de l'efficacité de la coloration et/ou de l'éclaircissement car les polymères à base de silicium résultant de cette réaction pénètrent peu dans la fibre du fait de leur taille, diminuant d'autant le pouvoir éclaircissant ou de coloration de la composition.

Ces buts et d'autres sont atteints par la présente invention qui a donc pour objet un procédé de coloration et/ou d'éclaircissement des fibres kératiniques humaines, dans lequel on met en contact lesdites fibres avec :
• une première composition avec une teneur en eau inférieure à 10% en poids et comprenant un ou plusieurs composés aminosiliciés de formule suivante (I) : 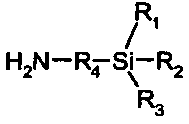 Formule dans laquelle :
   R₁, R₂, R₃ identiques ou différents désignent :
      - un radical alcoxy en C₁-C₂₀ linéaire ou ramifié, dont la partie alkyle est éventuellement interrompue par un ou plusieurs atomes d'oxygène, et plus particulièrement un radical alcoxy en C₁-C₂₀ linéaire ou ramifié, de préférence en C₁-C₄
      - un radical alcényloxy en C₂-C₂₀ linéaire ou ramifié de préférence en C₂-C₄
   R₄ est un radical divalent de structure :
   Dans laquelle:
   - R₆ identiques ou différents, désignent un radical alkyle en C₁-C₄ linéaire ou ramifié, de préférence méthyle ou éthyle, éventuellement substitué par un ou plusieurs groupements hydroxy, un radical NH₂, un radical hydroxy, un radical cyano, un radical Z₁₂NH₂, un radical Z₁₃NH Z₁₄NH₂, un radical alcényle en C₂-C₁₀ linéaire ou ramifié, de préférence en C₂-C₄, avec Z₁₂,Z₁₃ et Z₁₄ désignant indépendamment l'un de l'autre un radical alkylène linéaire en C₁-C₂₀ de préférence en C₁-C₁₀ mieux en C₁-C₄
   - désignent indépendamment l'un de l'autre, un radical alkylène linéaire en C₁-C₂₀
   - h vaut 0, 1, 2, 3,4 ou 5
   - a représente la liaison à l'atome de silicium
   - b représente la liaison à l'atome d'azote du groupement amino,
* une deuxième composition comprenant un ou plusieurs agents oxydants.

Elle a de même pour objet une composition avec une teneur en eau inférieure à 10% en poids comprenant un ou plusieurs composés aminosiliciés de formule(I) précitée et un ou plusieurs corps gras:

Un autre objet de l'invention est constitué par un dispositif à plusieurs compartiments comprenant dans au moins l'un d'eux une composition telle que précédemment, et dans au moins un autre, une composition oxydante.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Dans une variante, la composition présente une teneur en eau inférieure à 2 % en poids, et de préférence inférieure à 1% en poids par rapport au poids de ladite composition. Dans ce dernier cas on parle alors d'une composition substantiellement anhydre. Il est à noter qu'il s'agit plus particulièrement d'eau liée, comme l'eau de cristallisation des sels ou des traces d'eau absorbée par les matières premières utilisées dans la réalisation des compositions selon l'invention.

Il est également précisé, que compte tenu de l'usage qui en est fait, la composition selon l'invention ne comprend pas d'ingrédient qui la rendrait inutilisable pour la coloration et/ou l'éclaircissement des fibres kératinique humaines. Ainsi, les ingrédients qu'elle comprend sont cosmétiquement acceptables.

Comme indiqué précédemment, la composition anhydre (première composition) mise en oeuvre dans le procédé selon l'invention, comprend un ou plusieurs composés aminosiliciés de formule (I).

De préférence, dans la formule (I), R₁ et R₂ sont identiques.

Selon un mode particulièrement avantageux le composé de formule (I) ne comprend qu'un atome de silicium.

A titre d'exemples de composés de formule (I) convenant à la mise en oeuvre de l'invention, on peut citer les composés suivants

| | |
|---|---|
| | 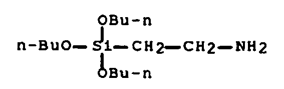 |
| 914384-30-8 Methanediamine, N-[7- (trimethoxysilyl)heptyl]- | 894393-40-9 Ethanamine, 2-(tributoxysilyl)- |
| 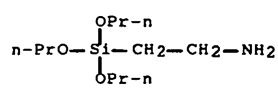 | 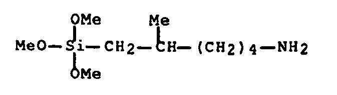 |
| 894393-33-0 Ethanamine, 2-(tripropoxysilyl)- | 852566-95-1 1-Hexanamine, 5-methyl-6-(trimethoxysilyl)- |
| 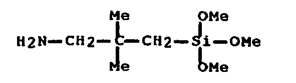 | |
| 848941-45-7 | |
| 1-Propanamine, 2,2-dimethyl-3-(trimethoxysilyl)- | |
| | 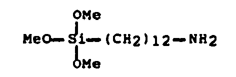 |
| | 771581-51-2 1-Dodecanamine, 12-(trimethoxysilyl)- |
| 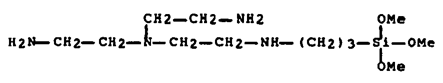 | |
| 610323-36-9 1,2-Ethanediamine, N,N-bis(2-aminoethyl)-N'-[3-(trimethoxysilyl)propyl]- | |
| 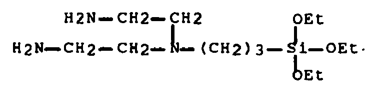 | 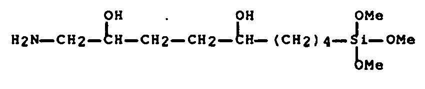 |
| 603111-49-5 1,2-Ethanediamine, N-(2-aminoethyl)-N-[3-(triethoxysilyl)propyl]- | 587877-12-1 2,5-Nonanediol, 1-amino-9-(trimethoxysilyl)- |
| 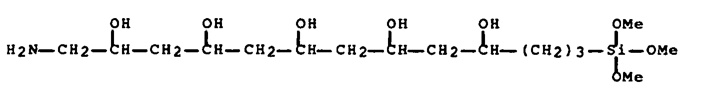 | |
| 587877-24-5 2,4,6,8,10-Tridecanepentol, 1-amino-13-(trimethoxysilyl) | |
| 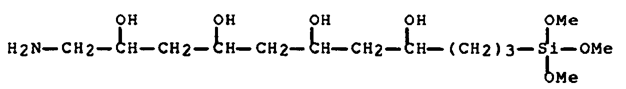 | |
| 587877-22-3 2,4,6,8-Undecanetetrol, 1-amine-11-(trimethoxysilyl) | |
| 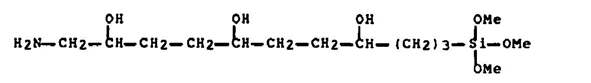 | |
| 587877-14-3 2,5,8-Undecanetriol, 1-amino-11-(trimethoxysilyl)- | |
| 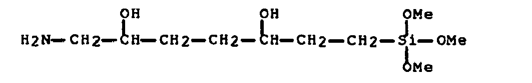 | |
| 587877-10-9 2,5-Heptanediol, 1-amino-7-(trimethoxysilyl)- | |
| 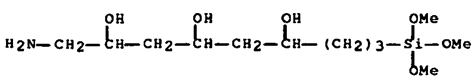 | 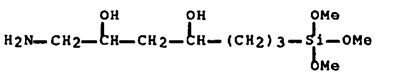 |
| 587877-08-5 2,4,6-Nonanetriol, 1-amino-9-(trimethoxysily)- | 587877-06-3 2,4-Heptanediol, 1-amino-7-(trimethoxysilyl)- |
| 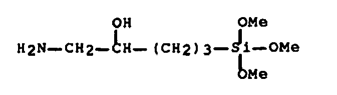 | 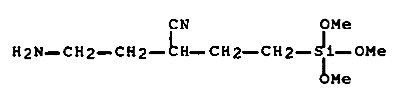 |
| 587877-04-1 2-Pentanol, 1-amino-5-(trimethoxysilyl)- | 587876-76-4 Butanenitrile, 2-(2-aminoethyl)-4-(trimethoxysilyl) |
| | 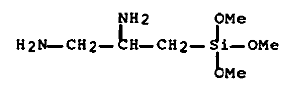 |
| 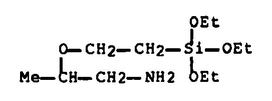 | 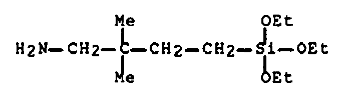 |
| 327024-70-4 1-Propanamine, 2-[2-(triethoxysilyl)ethoxy]- | 327024-67-9 4-Amino-3,3-dimethylbutyltriethoxysilane |
| 299199-34-1 1,2-Ethanediamine, N-(2-aminoethyl)-N'-[2-[[3-(triethoxysilyl)propyl]amino]ethyl] | |
| 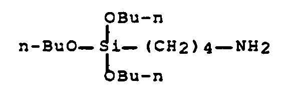 | |
| 314733-26-1 1-Butanamine, 4-(tributoxysilyl)- | |
| 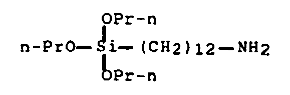 | 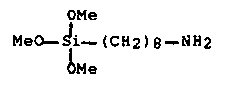 |
| 253596-69-9 1-Dodecanamine, 12-(tripropoxysilyl) | 253596-68-8 1-Octanamine, 8-(trimethoxysilyl) |
| | 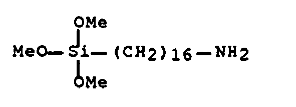 |
| | 193157-95-8 16-Aminohexadecyltrimethoxysilane, |
| | 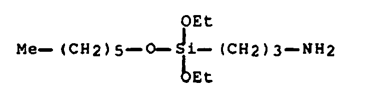 |
| | 183235-71-4 3-(Diethoxy(hexyloxy)silyl]-1-propanamine |
| 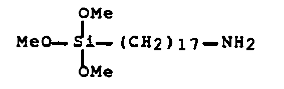 | 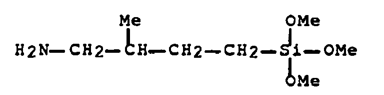 |
| 163193-89-3 (17-Aminoheptadecyl)trimethoxysilane | 157923-78-9 4-Amino-3-methylbutyltrimethoxysilane |
| 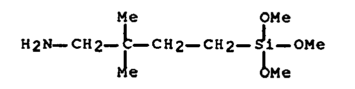 | |
| 157923-74-5 4-(Trimethoxysilyl)-2,2-dimethylbutanamine | |
| | 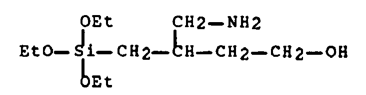 |
| | 143203-42-3 1-Butanol, 3-(aminomethyl)-4- |
| | 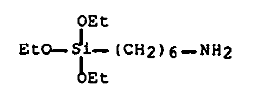 |
| | 134821-45-7 1-Hexanamine, 6-(triethoxysilyl)- |
| 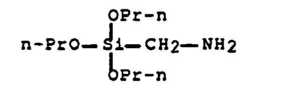 | |
| 131535-65-4 Methanamine, 1-(tripropoxysilyl)- | |
| | 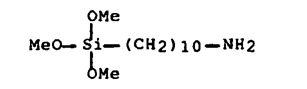 |
| | 120183-15-5 (10-Aminodecyl)trimethoxysilane |
| 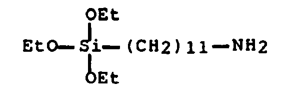 | |
| 116821-45-5 11-(Aminoundecyl)triethoxysilane | |
| | 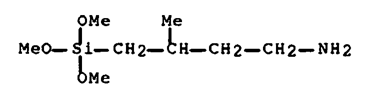 |
| | 106890-59-9 1-Butanamine, 3-methyl-4-(trimethoxysilyl) |
| | |
| 103526-27-8 N,N-Di(2-aminoethyl)-3-aminopropyltrimethoxysilane | |
| 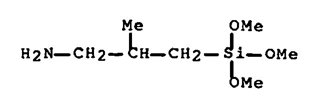 | |
| 99503-87-4 1-Propanamine, 2-methyl-3-(trimethoxysilyl)- | |
| 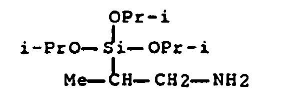 | 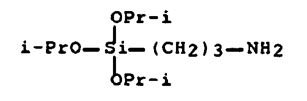 |
| 94989-07-8 1-Propanamine, 2-[tris(1-methylethoxy)silyl]- | 94989-06-7 3-Aminopropyltriisopropoxysilane |
| 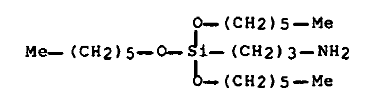 | 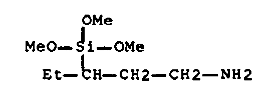 |
| 94277-92-6 3-[Tris(hexyloxy)silyl]-1-propanamine | 92116-16-0 3-(Trimethoxysilyl)-1-pentanamine |
| 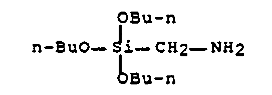 | |
| 84869-17-0 Aminomethyltributoxysilane | |
| 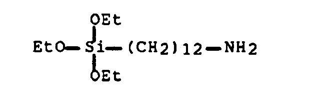 | 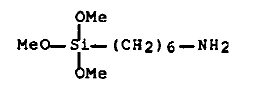 |
| 83943-65-1 1-Dodecanamine, 12-(triethoxysilyl) | 83943-64-0 1-Hexanamine, 6-(trimethoxysilyl) |
| | 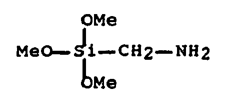 |
| | 71408-48-5 Methanamine, 1-(trimethoxysilyl)- |
| 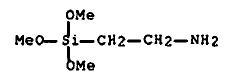 | 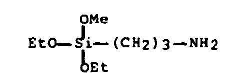 |
| 65644-31-7 2-(Trimethoxysilyl)ethylamine | 61083-96-3 1-Propanamine, 3-(diethoxymethoxysilyl)- |
| 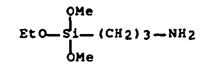 | |
| 61083-95-2 1-Propanamine, 3-(ethoxydimethoxysilyl) | |
| | 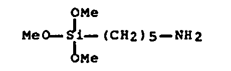 |
| | 54894-82-5 1-Pentanamine, 5-(trimethoxysilyl)- |
| | 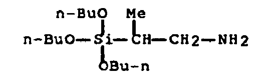 |
| | 53813-14-2 1-Propanamine, 2-(tributoxysilyl)- |
| | 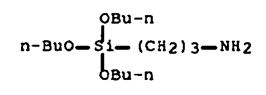 |
| | 52340-01-9 1-Propanamine, 3-(tributoxysilyl)- |
| 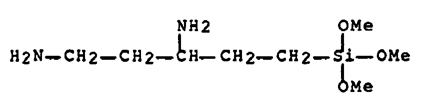 | 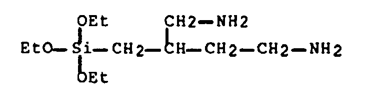 |
| 51279-08-4 1,3-Pentanediamine, 5-(trimethoxysilyl)- | 51279-07-3 1,4-Butanediamine, 2-[(triethoxysilyl)methyl]- |
| 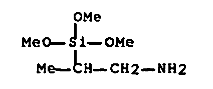 | |
| 50602-95-4 | |
| 1-Propanamine, 2-(trimethoxysilyl)- | |
| 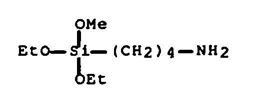 | 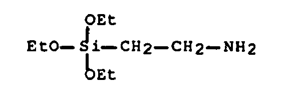 |
| 45116-10-7 1-Butanamine, 4-(diethoxymethoxysilyl)- | 45074-31-5 2-Aminoethyltriethoxysilane |
| 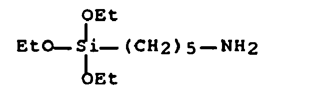 | |
| 1067-48-7 1-Pentanamine, 5-(triethoxysilyl)- | |
| | |
| 919-30-2 3-Aminopropyltriethoxysilane | |
| 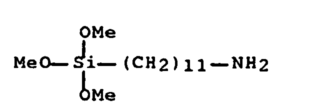 | 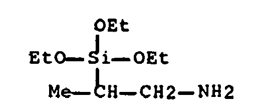 |
| 40762-31-0 11-Aminoundecyltrimethoxysilane | 36957-84-3 (2-Aminoisopropyl)triethoxysilane |
| 26092-76-2 Butylamine, 3-[3-(trimethoxysilyl)propoxy]-(8CL) | |
| | 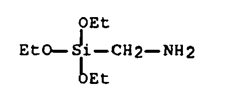 |
| | 18306-83-7 Aminomethyltriethoxysilane |
| | 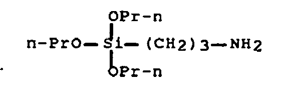 |
| | 18082-68-3 1-Propanamine, 3-(tripropoxysilyl) |
| 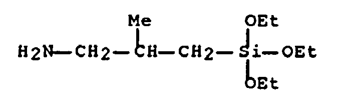 | |
| 17961-40-9 1-Propanamine, 2-methyl-3-(triethoxysilyl)- | |
| | 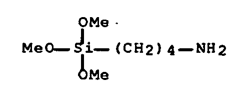 |
| | 15005-59-1 (4-Aminobutyl)trimethoxysilane |
| | 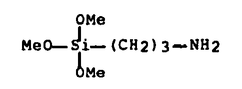 |
| | 13822-56-5 (3-Aminopropyl)trimethoxysilane |
| 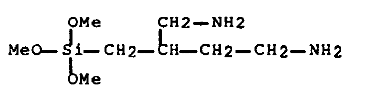 | 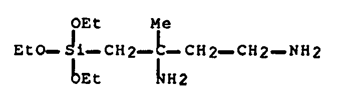 |
| 6037-49-6 1,4-Butanediamine, 2-[(trimethoxysilyl)methyl] | 5888-01-7 1,3-Butanediamine, 3-methyl-4-(triethoxysilyl) |
| 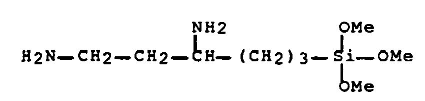 | 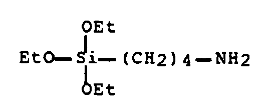 |
| 4543-14-0 1,3-Hexanediamine, 6-(trimethoxysilyl) | 3069-30-5 4-Aminobutyltriethoxysilane |

Les composés de formule (I) comprennent de préférence au moins un atome de silicium portant trois groupements alcoxy ou alcényloxy.

De préférence, dans la formule (I), R₁ et R₂ sont identiques.

Selon une autre variante, R₁, R₂, R₃ sont identiques.

Selon un mode de réalisation très avantageux de l'invention, le composé de formule (I) est le (3-aminopropyl)triéthoxysilane.

Habituellement, la teneur en composé de formule (I) représente de 0,1 à 50 % et de préférence de 1 à 30 %en poids par rapport au poids de la première composition.

La composition comprenant le ou les composés aminosiliciés de formule (I) peut également comprendre un ou plusieurs précurseurs de colorants d'oxydation, plus particulièrement une ou plusieurs bases d'oxydation éventuellement combinée(s) à un ou plusieurs-coupleurs ; un ou plusieurs colorants directs, ou leurs mélanges.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,Ngéthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-p-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N.N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine : la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-aminopyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yi)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-aminol-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

La composition peut éventuellement comprendre un ou plusieurs coupleurs choisis avantageusement parmi ceux conventionnellement utilisés pour la coloration des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(ß-hydroxyéthyl)amino-3,4-méthylène dioxybenzène, le 2,6-bis-(ß-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids de la composition, et de préférence de 0,005 à 5 % en poids par rapport au poids total de la composition.

La teneur en coupleur(s), s'il(s) est(sont) présent(s), représentent chacun avantageusement de 0,0001 à 10 % en poids par rapport au poids de la composition, et de préférence de 0,005 à 5 % en poids par rapport au poids total de la composition.

La composition avec le ou les composés aminosiliciés de formule (I) peut éventuellement comprendre un ou plusieurs colorants directs pouvant notamment être choisis parmi les espèces cationiques, neutres ou anioniques.

A titre d'exemple de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di- arylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quatemisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène : un groupement hydroxyle ; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Parmi les colorants directs benzéniques utilisables selon l'invention, on peut citer de manière non limitative les composés suivants :
- 1,4-diamino-2-nitrobenzène,
- 1-amino-2 nitro-4-ß- hydroxyéthylaminobenzène
- 1-amino-2 nitro-4-bis(β-hydroxyéthyl)-aminobenzène
- 1,4-bis(ß -hydroxyéthylamino)-2-nitrobenzène
- 1-ß-hydroxyéthylamino-2-nitro-4-bis-(β-hydroxyéthylamino)-benzène
- 1-ß-hydroxyéthylamino-2-nitro-4-aminobenzène
- 1-β-hydroxyéthylamino-2-nitro-4-(éthyl)(ß-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (II) à (V), et de préférence les composés de formules (II) et (IV) : 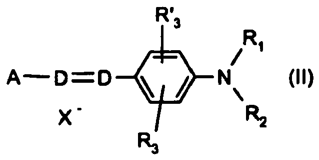 dans laquelle:
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄ , alcoxy en C₁-C₄ ou acétyloxy,
X⁻ représente un anion de préférence choisi parmi le chlorure, le méthyl sulfate et l'acétate,

A représente un groupement choisi par les structures A1 à A18 suivantes : 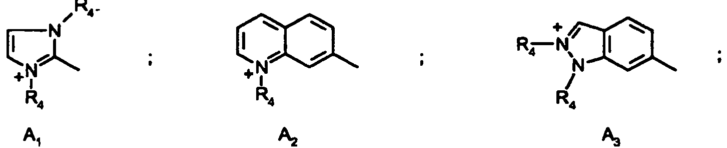 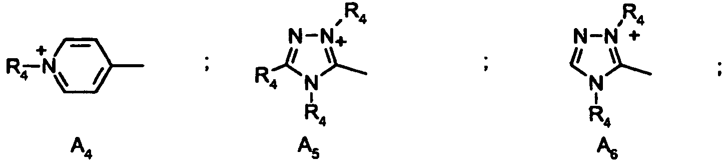 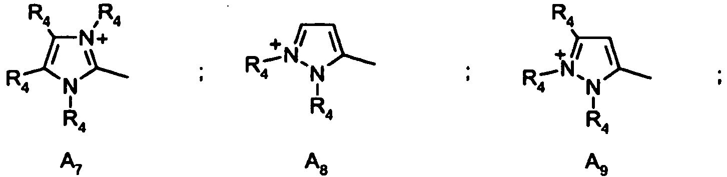 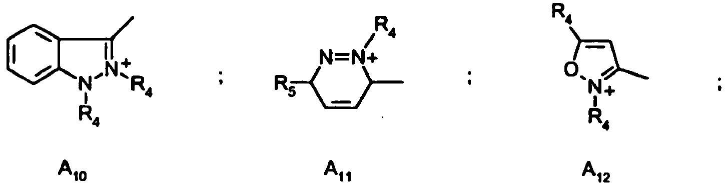 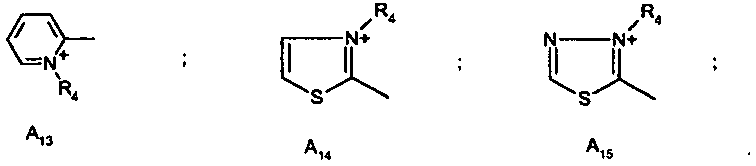 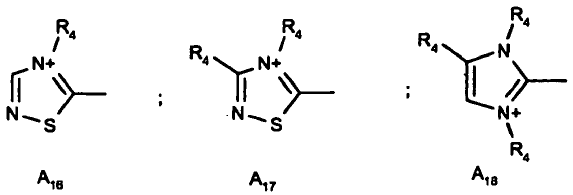 dans lesquelles R₄ représente un radical alkyle en C₁-C₄ pouvant être substitué par un radical hydroxyle et R₅ représente un radical alcoxy en C₁-C₄,; 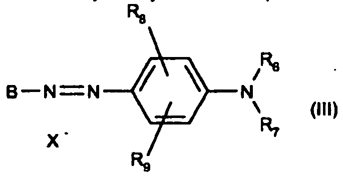 dans laquelle:
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X⁻ représente un anion de préférence choisi parmi le chlorure, le méthyl sulfate et l'acétate,
B représente un groupement choisi par les structures B1 à B6 suivantes : 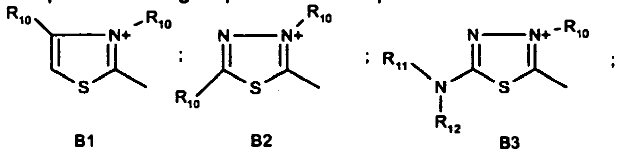 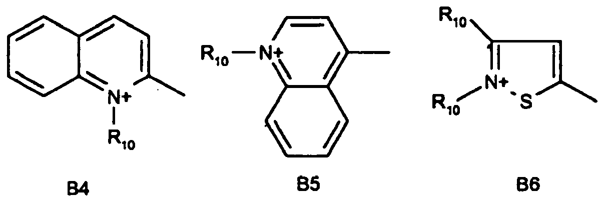 dans lesquelles R₁₀ représente un radical alkyle en C₁-C₄, R₁₁ et R₁₂, identiques ou différents, représentent un atome d'hydrogène ou un radical alkyle en C₁-C₄ ; 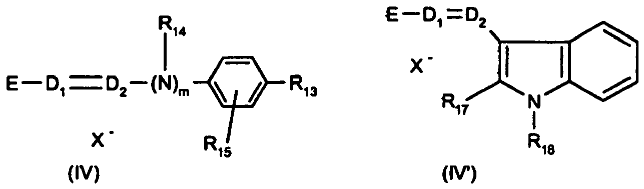
dans lesquelles :
R₁₃ représente un atome d'hydrogène, un radical alcoxy en C₁-C₄, un atome d'halogène tel que le brome, le chlore, l'iode ou le fluor,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH, m = 0 ou 1,
étant entendu que lorsque R₁₃ représente un groupement amino non substitué, alors D₁ et D₂ représentent simultanément un groupement -CH et m = 0,
X⁻ représente un anion de préférence choisi parmi le chlorure, le méthyl sulfate et l'acétate,
E représente un groupement choisi par les structures E1 à E8 suivantes : 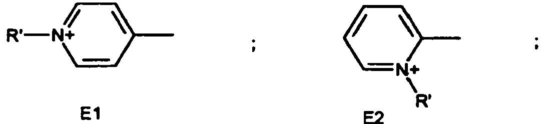 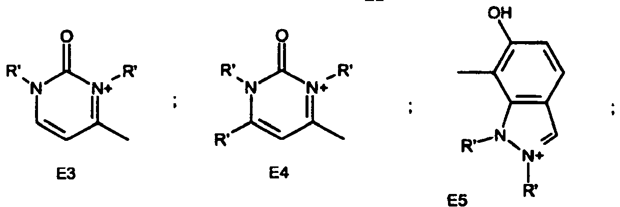 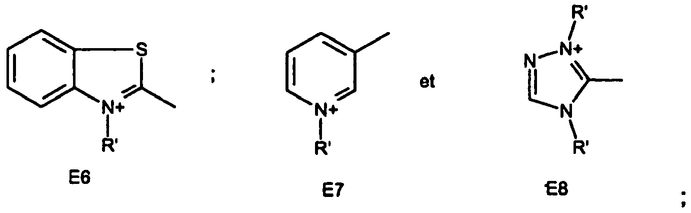 dans lesquelles R' représente un radical alkyle en C₁-C₄ ;
   lorsque m = 0 et que D₁ représente un atome d'azote, alors E peut également désigner un groupement de structure E9 suivante : 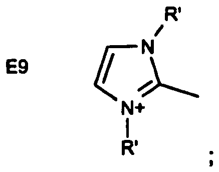 dans laquelle R' représente un radical alkyle en C₁-C₄.

   G-N=N-J (V)

   dans laquelle :

le symbole G représente un groupement choisi parmi les structures G, à G₃ suivantes : 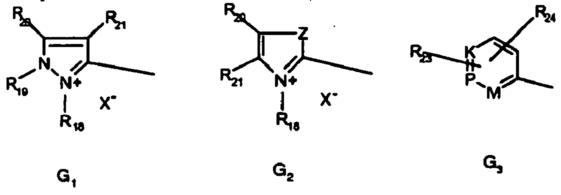 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂;
R₂₀ peut désigner en outre un atome d'hydrogène;
Z désigne un atome d'oxygène, de soufre ou un groupement -NR₁₉;
M représente un groupement -CH, -CR (R désignant alkyle en C₁-C₄),
ou -NR₂₂(X⁻)ᵣ;
K représente un groupement -CH, -CR (R désignant alkyle en C₁-C₄),
ou -NR₂₂(X⁻)ᵣ;
P représente un groupement -CH, -CR (R désignant alkyle en C₁-C₄),
ou -NR₂₂(X⁻)ᵣ; r désigne zéro ou 1;
R₂₂ représente un atome O⁻, un radical alcoxy en C₁-C₄, ou un radical alkyle en C₁-C₄; R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄ , un radical -NO₂;
X⁻ représente un anion de préférence choisi parmi le chlorure, l'iodure, le méthyl sulfate, l'éthyl sulfate, l'acétate et le perchlorate;
sous réserve que,
si R₂₂ désigne O⁻, alors r désigne zéro;
si K ou P ou M désignent -N-alkyle C₁-C₄ X⁻ , alors R₂₃ ou R₂₄ est ou non différent d'un atome d'hydrogène;
si K désigne -NR₂₂(X⁻)ᵣ, alors M= P= -CH, -CR;
si M désigne -NR₂₂(X⁻)ᵣ, alors K= P= -CH, -CR;
si P désigne -NR₂₂(X⁻)ᵣ, alors K= M et désignent -CH ou -CR;
si Z désigne un atome de soufre avec R₂₁ désignant alkyle en C₁-C₄ , alors R₂₀ est différent d'un atome d'hydrogène;
si Z désigne -NR₂₂ avec R₁₉ désignant alkyle en C₁-C₄, alors au moins l'un des radicaux R₁₈, R₂₀ ou R₂₁ du groupement de structure G₂ est différent d'un radical alkyle en C₁-C₄;
le symbole J représente :
- (a) un groupement de structure J₁ suivante : 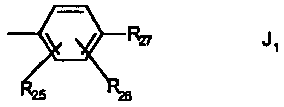 structure J₁ dans laquelle,
   R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
   R₂₆ représente un atome d'hydrogène, un atome d'halogène choisi parmi le chlore, le brome, l'iode et le fluor, un radical alkyle en C₁-C₄, alcoxy en C₁-C₄,
   ou forme avec R₂₇ ou R₂₈ un cycle à 5 ou 6 chaînons contenant ou non un ou plusieurs hétéroatomes choisis parmi l'azote, l'oxygène ou le soufre;
   R₂₇ représente un atome d'hydrogène, un radical -OH, un radical -NHR₂₈, un radical - NR₂₉R₃₀;
   R₂₈ représente un atome d'hydrogène, un radical alkyle en C₁-C₄, un radical monohydroxyalkyle en C₁-C₄, polyhydroxyalkyle en C₂-C₄, un radical phényle;
   R₂₉ et R₃₀, identiques ou différents, représentent un radical alkyle en C₁-C₄, un radical monohydroxyalkyle en C₁-C₄, polyhydroxyalkyle en C₂-C₄;
- (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
   et notamment un groupement de structure J₂ suivante : 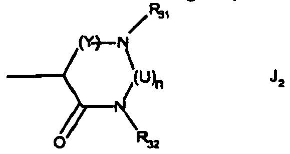 structure J₂ dans laquelle,
   R₃₁ et R₃₂, identiques ou différents, représentent un atome d'hydrogène , un radical alkyle en C₁-C₄, un radical phényle;
   Y désigne le radical -CO- ou le radical 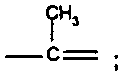
n = 0 ou 1, avec, lorsque n désigne 1, U désigne le radical -CO- .

Dans les structures (II) à (V) définies ci-dessus le groupement alkyle ou alcoxy en C₁-C₄ désigne de préférence méthyle, éthyle, butyle, méthoxy, éthoxy.

Parmi les composés de formules (II) et (IV), on préfère les composés suivants :

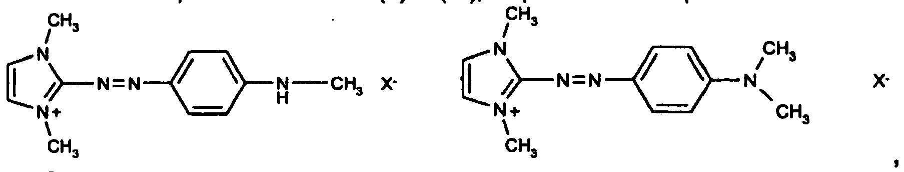

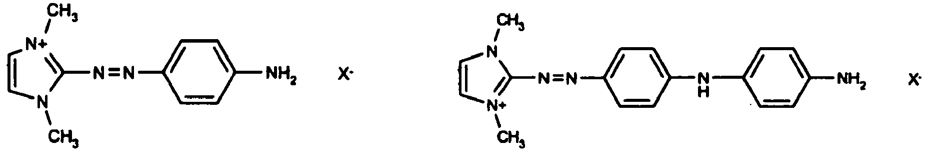

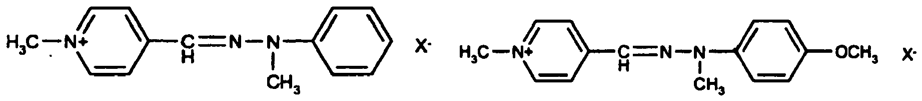

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
   ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous, An étant défini comme précédemment :

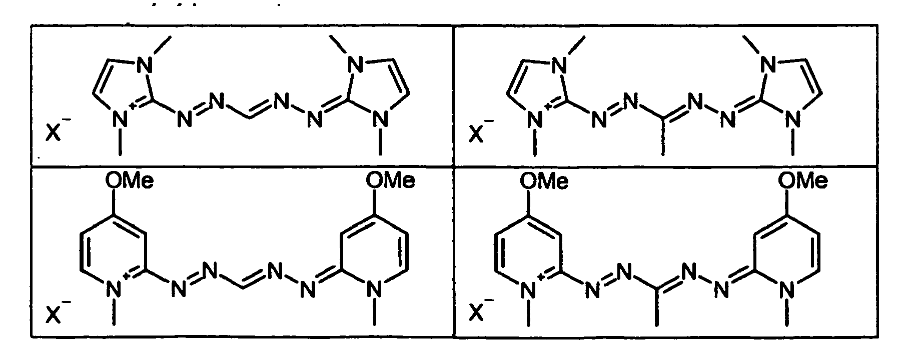

--

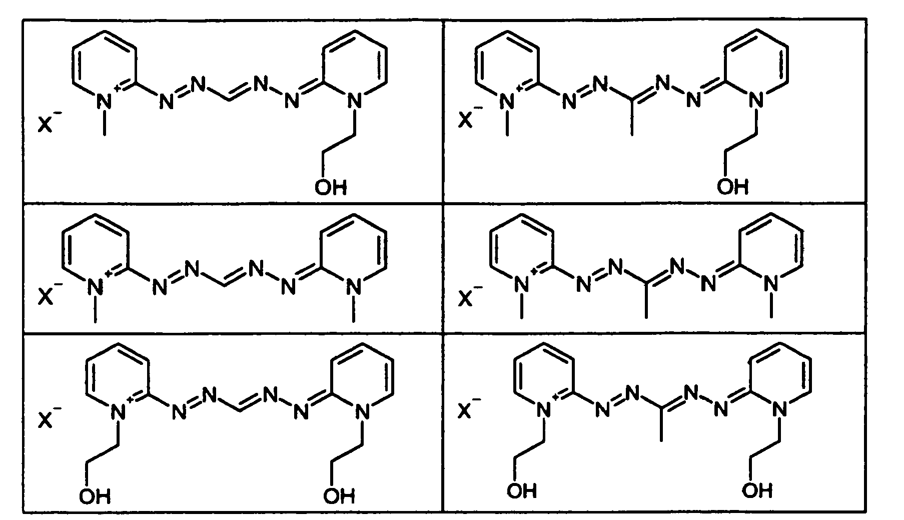

X⁻ représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quatemisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quatemisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Ces polychromophores sont reliés entre eux au moyen d'au moins un bras de liaison comprenant éventuellement au moins un atome d'azote quatemisé engagé ou non dans un hétérocycle saturé ou non, éventuellement aromatique.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quatemisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quatemisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent plus particulièrement de 0,0001 à 10 % en poids par rapport au poids total de la composition, et de préférence de 0,005 à 5 % en poids par rapport au poids total de la composition.

De préférence, les colorants directs mis en oeuvre sont des colorants directs neutres ou cationiques.

Conformément à un mode de réalisation particulier de l'invention, la composition comprenant le ou les composés aminosiliciés de formule (I) comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0.1%). En outre, ces composés organiques possèdent de préférence des propriétés lubrifiantes. Notamment, au sens de la présente invention, un corps gras est un composé choisi parmi un alcool gras, un acide gras, un ester d'acide gras, un ester d'alcool gras, une huile minérale, végétale, animale ou synthétique, une silicone ou une cire. Il est rappelé qu'au sens de l'invention, les alcools, esters et acides gras présentent au moins un groupement hydrocarboné, linéaire ou ramifié, saturé ou insaturé, comprenant 6 à 30 atomes de carbone, éventuellement substitué, en particulier par un ou plusieurs groupements hydroxyle (en particulier 1 à 4). S'ils sont insaturés, ces composés peuvent comprendre une à trois double-liaisons carbone-carbone, conjuguées ou non.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 6 à 30) atomes de carbone comme les triglycérides des acides heptandique ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que Parléam^{®} ; les isoparaffines comme l'isohexadécane et l'isodécane.
- les alcools gras sont saturés ou insaturés, linéaires ou ramifiés, et comportent de 8 à 30 atomes de carbone, on peut citer l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ; comme huiles fluorées, on peut citer aussi le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

La cire ou les cires sont choisies notamment, parmi la cire de Camauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les acides gras peuvent être saturés ou insaturés et comportent de 6 à 30 atomes de carbone, en particulier de 9 à 30 atomes de carbone. Ils sont choisis plus particulièrement parmi l'acide myristique, l'acide palmitique, l'acide stéarique, l'acide béhénique, l'acide oléïque, l'acide linoléique, l'acide linolénique et l'acide isostéarique,

Les esters sont les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total de carbone des esters étant supérieur ou égal à 10.

Parmi les monoesters , on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-héxyle, le palmitate de 2-octyldécyle, les myristates d'alkyles tels que le myristate d'isopropyle, de butyle, de cétyle, de 2-octyldodécyle, de mirystyle, de stéaryle le stéarate d'hexyle, le stéarate de butyle, le stéarate d'isobutyle ; le malate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de dlisopropyle l'adipate de d'isopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; trilactate de glycéryle ; trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-héxyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le malate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou dioléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

Les silicones utilisables dans les compositions cosmétiques de la présente invention, sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1 m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modfiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcydotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE^{®} FZ 3109 commercialisée par la société UNION CARBIDE, de formule: avec D" :

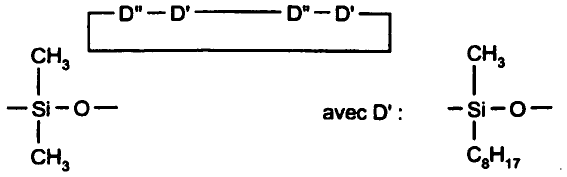

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASILE® commercialisées par la société "RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :

R₂SiO_{2/2}, R₃SiO_{1/2}, RSiO_{3/2} et SiO_{4/2}

dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre, les silicones décrites ci-dessus les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyllméthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET® L 722, L 7500, L 77, L 711 de la société UNION CARBIDE et l'alkyl (C₁₂)-méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le corps gras est un composé liquide à la température de 25°C et à la pression atmosphérique.

De préférence c'est un ester d'acide gras ou de l'huile de vaseline.

La composition comprenant le ou les composés aminosiliciés selon l'invention présente une teneur en corps gras comprise avantageusement entre 10 et 99% en poids, par rapport au poids de la composition, de préférence entre 20 et 90% en poids, préférentiellement entre 25 et 80% mieux entre 30 et 70% en poids.

Selon un mode de réalisation particulier de l'invention, cette composition comprend un ou plusieurs agents tensioactifs ; ces agents tensioactifs pouvant être non ioniques, anioniques, cationiques ou amphotères.

De préférence la composition comprend un ou plusieurs tensioactifs anioniques, plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Le tensioactif ou le mélange de tensioactifs, lorsqu'il est(sont) présent(s), représente(nt)plus particulièrement de 0,01 % et 60 % en poids par rapport au poids de la composition, de préférence entre 0,5 % et 50 % en poids et encore plus préférentiellement entre 1 % et 40 % en poids mieux entre 4 et 30% en poids.

La composition comprenant le ou les composés aminosiliciés de formule (I) peut encore comprendre un agent alcalinisant différent du ou des dits composés aminosiliciés.

Parmi les agents alcalinisants on peut citer, à titre d'exemple, seuls ou en mélanges, l'ammoniaque ; les composés de formule suivante : 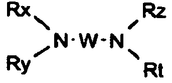 dans laquelle W est un reste propylène éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆;

De préférence, l'agent alcalinisant supplémentaire, s'il est présent, n'est pas de l'ammoniaque. Cet agent peut permettre de régler le pH de la composition appliquée sur cheveux ou tout du moins de sa partie aqueuse si elle est présente. Ce pH est de préférence compris entre 4 et 11 et encore plus préférentiellement entre 7 et 10.5. Si l'ammoniaque est employé en tant qu'agent alcalinisant supplémentaire, alors de préférence, sa teneur est inférieure ou égale à 0,03 % en poids de la composition finale (exprimée en NH₃), plus particulièrement inférieure ou égale à 0,01 % en poids par rapport à la composition finale. Il est rappelé que la composition finale résulte du mélange de la composition contenant le ou les composés de formule (i) avec de la composition oxydante ; ce mélange étant réalisé soit avant application sur les fibres kératiniques (préparation extemporanée) soit directement sur les fibres kératiniques (application successive séparée, sans rinçage intermédiaire, des compositions sur les fibres kératiniques). De préférence, l'ammoniaque n'est pas employé comme agent alcalinisant supplémentaire.

La composition comprenant le ou les composés aminosiliciés de formule (I) peut également renfermer divers adjuvants utilisés classiquement dans les compositions pour la coloration des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20 % en poids par rapport au poids de la composition.

La composition selon l'invention peut résulter du mélange extemporané du ou des composés aminosiliciés de l'invention avec le reste de la composition contenant moins de 10% d'eau.

Le procédé de coloration selon l'invention est mis en oeuvre en appliquant sur les fibres kératiniques, sèches ou humides, la composition telle que définie précédemment ou en la mélangeant extemporanément ou sur le cheveu avec une composition aqueuse.

Selon le procédé conforme à l'invention, la composition est mise en contact avec une composition comprenant un ou plusieurs agents oxydants (composition oxydante).

Plus particulièrement, la composition oxydante est aqueuse et comprend éventuellement un ou plusieurs solvants organiques.

A titre de solvant organique, on peut par exemple citer, les alcanols, linéaires ou ramifiés, en C₂-C₄, tels que l'éthanol et l'isopropanol ; le glycérol ; les glycols et éthers glycols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants peuvent être présents dans des proportions allant habituellement de 1 à 40 % en poids par rapport au poids de la composition oxydante, et de préférence de 5 à 30 % en poids.

Plus particulièrement, l'agent oxydant est choisi parmi le peroxyde d'hydrogène ; le peroxyde d'urée ; les bromates ou ferricyanures de métaux alcalins ; les sels peroxygénés comme par exemple les persulfates, les perborates et les percarbonates de métaux alcalins ou alcalino-terreux, comme le sodium, le potassium, le magnésium ; ou leurs mélanges.

Cet agent oxydant est avantageusement constitué du peroxyde d'hydrogène et notamment par une solution aqueuse dont le titre peut varier, plus particulièrement, de 1 à 40 volumes, et encore plus préférentiellement de 5 à 40 volumes.

La composition oxydante peut également comprendre au moins un agent alcalinisant et/ou au moins un agent acidifiant. De préférence la composition oxydante contient au moins un agent acidifiant.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante est inférieur à 7.

La composition oxydante peut se présenter sous la forme d'une solution, une émulsion ou un gel.

La composition oxydante peut également renfermer d'autres ingrédients classiquement employés dans le domaine, comme notamment ceux détaillés auparavant dans le cadre de la composition comprenant le ou les composés aminosiliciés de formule (I).

Selon un mode de réalisation particulier de l'invention, la quantité de composition oxydante par rapport à celle de la composition comprenant le ou le composés organosiliciés, est telle que la teneur en composé organosilicié de formule (I) soit comprise entre 2 et 8 % en poids dans la composition finale. On rappelle que par composition finale, on définit la composition résultant du mélange de la composition contenant le ou les composés de formule (I) avec de la composition oxydante ; ce mélange étant réalisé soit avant application sur les fibres kératiniques (préparation extemporanée) soit directement sur les fibres kératiniques (application successive séparée, sans rinçage intermédiaire, des compositions sur les fibres kératiniques).

Selon un premier mode de réalisation de l'invention, le procédé est mis en oeuvre en appliquant sur les fibres kératiniques, sèches ou humides, une composition obtenue par mélange extemporané, au moment de l'emploi, de la composition comprenant le ou les composés aminosiliciés de formule (I). (première composition) et de la composition oxydante (deuxième composition).

Selon un deuxième mode de réalisation de l'invention, le procédé est mis en oeuvre en appliquant les deux compositions sur les fibres kératiniques, sèches ou humides, successivement et sans rinçage intermédiaire.

En particulier, le procédé est mis en oeuvre en appliquant sur les fibres kératiniques, sèches ou humides, successivement et sans rinçage intermédiaire, notamment à l'eau, la composition comprenant le ou les composés aminosiliciés de formule (I) (première composition) puis la composition oxydante (deuxième composition).

Selon une autre possibilité, le procédé est mis en oeuvre en appliquant sur les fibres kératiniques, sèches ou humides, successivement et sans rinçage intermédiaire, notamment à l'eau, la composition oxydante (deuxième composition) puis la composition comprenant le ou les composés aminosiliciés de formule (I). (première composition).

Quelle que soit la variante du procédé retenue, le mélange présent sur les fibres (résultant soit du mélange extemporané ou bien résultant de l'application successive de la composition comprenant un ou plusieurs composés aminosiliciés de formule (I) et de la composition oxydante) est laissé en place pour une durée, en général, de l'ordre de 1 minute à 1 heure, de préférence de 10 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, lavées au shampooing, rincées à nouveau à l'eau puis séchées ou laissées à sécher.

Un autre objet de l'invention est constitué par une composition avec une teneur en eau inférieure à 10 % en poids, comprenant un ou plusieurs aminoalcoxysilanes de formule (I) détaillée auparavant associé à un ou plusieurs corps gras.

De préférence, la composition comprend une teneur en eau inférieure ou égale à 2% en poids, plus avantageusement inférieure à 1% en poids.

De préférence, la composition ne comprend pas d'eau liée, comme l'eau de cristallisation des sels ou des traces d'eau absorbée par les matières premières utilisées dans la réalisation des compositions selon l'invention.

Tout ce qui a été détaillé précédemment concernant la composition comprenant le ou les composés aminosiliciés de formule (I) reste applicable et ne sera donc pas repris dans cette partie de la description.

Enfin, l'invention concerne un dispositif à plusieurs compartiments comprenant dans au moins l'un d'eux une composition comprenant un ou plusieurs composés aminosiliciés de formule (I) telle que définie précédemment, et dans au moins un autre, une composition comprenant un ou plusieurs agents oxydants décrite également auparavant.

Les exemples suivants servent à illustrer l'invention sans toutefois présenter un caractère limitatif.

### EXEMPLES

### Exemple 1 : Compositions éclaircissantes

### 1. Les compositions selon l'invention

### (les teneurs sont exprimées en g%)

| | Composition 1 | Composition 2 |
|---|---|---|
| Lauryl sulfate de sodium en poudre | 11.25 | 10 |
| Talc | 33.75 | 30 |
| Myristate d'isopropyle | 45 | 40 |
| (3-aminopropyl)triéthoxysilane (1) | 10 | 0 |
| (3-aminopropyl)tris[2-(2-méthoxy-éthoxy]silane (2) | 0 | 20 |

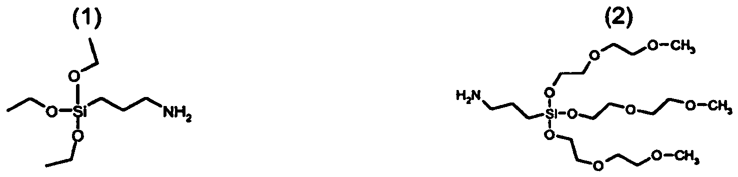

### 2. Composition aqueuse à base d'ammoniaque hors invention

### (les teneurs sont exprimées en g%)

| | |
|---|---|
| Alcool oléique polyglycérolé à 2 moles de glycérol | 4 |
| Alcool oléique polyglycérolé à 4 moles de glycérol | 5.69 MA (*) |
| Acide oléique | 3 |
| Amine oléique à 2 moles d'oxyde d'éthylène (Ethomeen 012 ; Akzo) | 7 |
| Laurylamino succinamate de diéthylaminopropyle, sel de sodium à 55% de matières active | 3.0 MA (*) |
| Alcool oléique | 5 |
| Diethanolamide d'acide oléique | 12 |
| Alcool éthylique | 7 |
| Propylène glycol | 3.5 |
| Dipropylèneglycol | 0.5 |
| Monométhylether de propylèneglycol | 9 |
| Acétate d'ammonium | 0.8 |
| Ammoniaque à 20 % | 10 |
| Eau déminéralisée q.s.p. | 100 g |

| | |
|---|---|
| (*) MA = matière active | |

### 3. Procédé

Au moment de l'emploi, chaque composition est mélangée poids pour poids avec une solution aqueuse de peroxyde d'hydrogène à 20 volumes à pH2,3.

Le pH des solutions résultantes est de 10,1 ± 0,1.

Chaque mélange est ensuite appliqué sur une mèche châtain (hauteur de ton 5) naturelle.

Le temps de pose est de 30 minutes à température ambiante.

A l'issue de ce temps, les mèches sont traitées pendant une minute dans une solution hydro-alcoolique dont la composition est indiquée ci-dessous (quantité exprimées en g%):

| | |
|---|---|
| Alcool benzylique | 5 |
| Ethanol dénaturé | 15 |
| Hydroxyéthyl oléyl dimonium chloride à 70MA | 5 |
| Acide benzoïque | 0.2 |
| Eau déminéralisée | Qsp 100 |

Les mèches sont ensuite lavés avec un shampooing Elsève multivitamines ®, rincées à l'eau puis mises à sécher.

### 4. Résultats

On évalue l'éclaircissement des mèches par des mesures colorimétriques au moyen d'un colorimètre MINOLTA CM2002.

| | L* | a* | b* | Eclaircissement (ΔE) |
|---|---|---|---|---|
| Mèche non traitée | 22.97 | 3.40 | 4.25 | / |
| Composition de l'invention 1 | 25.83 | 5.94 | 7.85 | 5.25 |
| Composition de l'invention 2 | 27.51 | 6.12 | 8.59 | 6.85 |
| Composition de l'état de l'art | 26.40 | 6.47 | 8.64 | 6.36 |

On constate que les compositions selon l'invention permettent d'éclaircir de manière similaire à la composition hors invention alors qu'elles ne présentent pas les inconvénients de celle-ci, dus à la présence d'ammoniaque (pas de différence significative).

### Exemple 2 : composition éclaircissante contenant des colorants directs

On prépare la composition de l'invention suivante :

### (les teneurs sont exprimées en g%)

| | Formule 1 | Formule 2 |
|---|---|---|
| Kaolin | 25 | 25 |
| Sodium carboxymethyl amidon | 12 | 12 |
| Myristate d'isopropyle | 48,25 | 48,25 |
| Silice pyrogénée | 2,25 | 2,25 |
| Sodium lauryl éther sulfate à 70% dans l'eau (Texapon AOS 225 UP de COGNIS) | 12,5 | 12,5 |
| Disperse red 17 | 0.99 | - |
| Basic blue 99 | - | 1.35 |
| (3-aminopropyl)triethoxysilane | 10 | 10 |

Deux formules sont préparées, l'une à base de colorant hydrophile et l'autre à base de colorant hydrophobe.

On procède comme pour l'exemple précédent.

La formule 1 conduit à un rouge violine puissant.

La formule 2 donne un violet puissant.

## Revendications

1. Procédé de coloration et/ou d'éclaircissement des fibres kératiniques humaines, dans lequel on met en contact lesdites fibres avec :
*une première composition avec une teneur en eau inférieure à 10% en poids et comprenant un ou plusieurs composés aminosiliciés de formule suivante (I) : 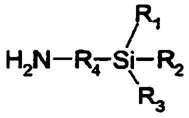 Formule dans laquelle:
R₁, R₂, R₃ identiques ou différents désignent :
- un radical alcoxy en C₁-C₂₀ linéaire ou ramifié, dont la partie alkyle est éventuellement interrompue par un ou plusieurs atomes d'oxygène,
- un radical alcényloxy en C₂-C₂₀ linéaire ou ramifié de préférence en C₂-C₄
R₄ est un radical divalent de structure : 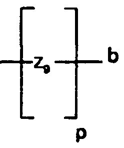 Dans laquelle:
■ Z₉ désigne un radical alkylène linéaire en C₁-C₂₀
■ p vaut 1
■ a représente la liaison à l'atome de silicium
■ b représente la liaison à l'atome d'azote du groupement amino,
* une deuxième composition comprenant un ou plusieurs agents oxydants.

2. Procédé selon la revendication précédente, **caractérisé en ce que** le composé de formule (I) est tel que R₁ et R₂ sont identiques.

3. Procédé selon l'une quelconque des revendications précédentes, **caractérisé en ce que** le composé de formule (I) est tel que R₁, R₂, R₃ sont identiques.

4. Procédé selon: l'une quelconque des revendications précédentes, **caractérisé en ce que** la teneur en composé de formule (I) représente de 0,1 à 50 % en poids par rapport au poids de la première composition.

5. Procédé selon l'une quelconque des revendications précédentes, **caractérisé en ce que** la première composition comprend un ou plusieurs précurseurs de colorant d'oxydation choisis parmi les bases et les coupleurs d'oxydation ; un ou plusieurs colorants directs, ou leurs mélanges.

6. Procédé selon l'une quelconque des revendications précédentes, **caractérisé en ce que** la première composition comprend un ou plusieurs corps gras.

7. Procédé selon la revendication précédente, **caractérisée en ce que** le corps gras est un composé choisi parmi un alcool gras, un acide gras, un ester d'acide gras, un ester d'alcool gras, une huile minérale, végétale, animale ou synthétique, une silicone ou une cire.

8. Procédé selon l'une quelconque des revendications 6 ou 7, **caractérisée en ce que** le corps gras est un ester d'acide gras ou de l'huile de vaseline.

9. Procédé selon l'une des revendications 6 à 8, **caractérisée en ce que** la teneur en corps gras est comprise entre 10 et 99 % en poids, par rapport au poids de la composition.

10. Procédé selon l'une quelconque des revendications précédentes, **caractérisé en ce que** l'on applique sur les fibres kératiniques, une composition obtenue par mélange extempotané, au moment de l'emploi, des première et deuxième compositions.

11. Procédé selon l'une quelconque des revendications 1 à 9, caractériséen ce que l'on applique les deux compositions sur les fibres kératiniques, successivement et sans rinçage intermédiaire.

12. Composition avec une teneur en eau inférieure à 10% en poids et comprenant un ou plusieurs composés aminosiliciés de formule suivante (I): 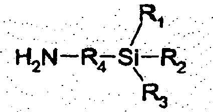 Formule dans laquelle :
R₁, R₂, R₃ identiques ou différents désignent :
- un radical alcoxy en C₁-C₂₀ linéaire ou ramifié, dont la partie alkyle est éventuellement interrompue par un ou plusieurs atomes d'oxygène,
- un radical alcényloxy en C₂-C₂₀ linéaire ou ramifié de préférence en C₂-C₄
R₄ est un radical divalent de structure : 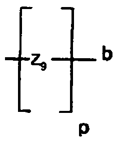 Dans laquelle :
■ Z₉, désign, un radical alkylène linéaire en C₁-C₂₀
■ p vaut 1
■ a représente la liaison à l'atome de silicium
■ b représente la liaison à l'atome d'azote du groupement amino.
Et un ou plusieurs corps gras.

13. Dispositif à plusieurs compartiments comprenant dans au moins l'un d'eux une composition telle que définie dans la revendication 12, et dans au moins un autre, une composition comprenant un ou plusieurs agents oxydants.

## Claims

1. Method of colouring and/or lightening human keratin fibres, in which said fibres are contacted with:
* a first composition having a water content of less than 10% by weight and comprising one or more aminosilicon compounds of formula (I) below: 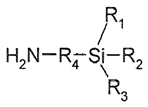 in which:
R₁, R₂ and R₃, which are identical or different, denote:
- a linear or branched C₁-C₂₀ alkoxy radical in which the alkyl moiety is optionally interrupted by one or more oxygen atoms,
- a linear or branched C₂-C₂₀, preferably C₂-C₄, alkenyloxy radical,
R₄ is a divalent radical of structure: 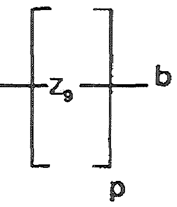
in which:
■ Z₉ denotes a C₁-C₂₀ linear alkylene radical
■ p is 1
■ a represents the bond to the silicon atom
■ b represents the bond to the nitrogen atom of the amino group,
* a second composition comprising one or more oxidizing agents.

2. Method according to the preceding claim, **characterized in that** the compound of formula (I) is such that R₁ and R₂ are identical.

3. Method according to either of the preceding claims, **characterized in that** the compound of formula (I) is such that R₁, R₂ and R₃ are identical.

4. Method according to any of the preceding claims, **characterized in that** the amount of compound(s) of formula (I) represents from 0.1% to 50% by weight, relative to the weight of the first composition.

5. Method according to any of the preceding claims, **characterized in that** the first composition comprises one or more oxidation dye precursors selected from oxidation couplers and bases, one or more direct dyes, or mixtures thereof.

6. Method according to any of the preceding claims, **characterized in that** the first composition comprises one or more fats.

7. Method according to the preceding claim, **characterized in that** the fat is a compound selected from a fatty alcohol, a fatty acid, a fatty acid ester, a fatty alcohol ester, a mineral, vegetable, animal or synthetic toil, a silicone or a wax.

8. Method according to either of Claims 6 and 7, **characterized in that** the fat is a fatty acid ester or liquid petrolatum.

9. Method according to any one of Claims 6 to 8, **characterized in that** the amount of fat is between 10% and 99% by weight, relative to the weight of the composition.

10. Method according to any of the preceding claims, **characterized in that** a composition obtained by extemporaneous mixing at the time of use of the first and second compositions is applied to the keratin fibres.

11. Method according to any of Claims 1 to 9 **characterized in that** the two compositions are applied to the keratin fibres successively and without intermediate rinsing.

12. Composition having a water content of less than 10% be weight and comprising one or more aminosilicon compound(s) of formula (I) below: 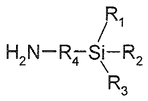 in which:
R₁, R₂ and R₃, which are identical or different, denote:
- a linear or branched C₁-C₂₀ alkoxy radical in which the alkyl moiety is optionally interrupted by one or more oxygen atoms,
- a linear or branched C₂-C₂₀ preferably C₂-C₄, alkenyloxy radical,
R₄ is a divalent radical of structure: 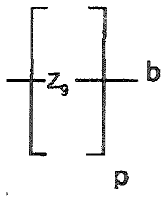
in which:
■ Z₉ denotes a C₁-C₂₀ linear alkylene radical
■ p is 1
■ a represents the bond to the silicon atom
■ b represents the bond to the nitrogen atom of the amino group,
and one or more fats.

13. Multiple-compartment device comprising, in at least one compartment, a composition as defined in Claim 12, and, in at least one other, a composition comprising one or more oxidizing agents.

## Patentansprüche

1. Verfahren zum Färben und/oder Aufhellen von menschlichen Keratinfasern, bei dem man die Fasern mit
*einer ersten Zusammensetzung, die einen Wassergehalt von weniger als 10 Gew.-% aufweist und eine oder mehrere Aminosiliciumverbindungen der folgenden Formel (I): 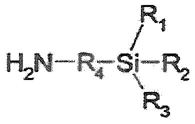 worin:
R₁, R₂ und R₃ gleich oder verschieden sind und für:
- einen linearen oder verzweigten C₁-C₂₀-Alkoxyrest, dessen Alkylteil gegebenenfalls durch ein oder mehrere Sauerstoffatome unterbrochen ist,
- einen linearen oder verzweigten C₂-C₂₀- und vorzugsweise C₂-C₄-Alkenyloxyrest
stehen,
R₄ für einen zweiwertigen Rest der Formel: 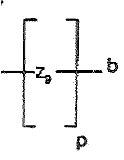
worin:
■ Z₉ für einen linearen C₁-C₂₀-Alkylenrest steht,
■ p gleich 1 ist,
■ a für die Bindung an das Siliciumatom steht,
■ b für die Bindung an das Stickstoffatom der Aminogruppe steht,
steht, umfasst,
*einer zweiten Zusammensetzung, die ein oder mehrere Oxidationsmittel umfasst,
in Berührung bringt.

2. Verfahren nach dem vorhergehenden Anspruch, **dadurch gekennzeichnet, dass** die Verbindung der Formel (I) so beschaffen ist, dass R₁ und R₂ gleich sind.

3. Verfahren nach einem der vorhergehenden Ansprüche, **dadurch gekennzeichnet, dass** die Verbindung der Formel (I) so beschaffen ist, dass R₁, R₂ und R₃ gleich sind.

4. Verfahren nach einem der vorhergehenden Ansprüche, **dadurch gekennzeichnet, dass** der Gehalt an Verbindung der Formel (I) 0,1 bis 50 Gew.-%, bezogen auf das Gewicht der ersten Zusammensetzung, beträgt.

5. Verfahren nach einem der vorhergehenden Ansprüche, **dadurch gekennzeichnet, dass** die erste Zusammensetzung eine oder mehrere Oxidationsfarbstoffvorstufen, die unter Oxidationsbasen und Oxidationskupplern ausgewählt sind, einen oder mehrere Direktfarbstoffe oder Mischungen davon umfasst.

6. Verfahren nach einem der vorhergehenden Ansprüche, **dadurch gekennzeichnet, dass** die erste Zusammensetzung einen oder mehrere Fettstoffe umfasst.

7. Verfahren nach dem vorhergehenden Anspruch, **dadurch gekennzeichnet, dass** es sich bei dem Fettstoff um eine unter einem Fettalkohol, einer Fettsäure, einem Fettsäureester, einem Fettalkoholester, einem Mineralöl, einem pfanzlichen Öl, einem tierischen Öl, einem synthetischen Öl, einem Silikon oder einem Wachs ausgewählte Verbindung handelt.

8. Verfahren nach einem der Ansprüche 6 bis 7, **dadurch gekennzeichnet, dass** es sich bei dem Fettstoff um einen Fettsäureeester oder Vaselineöl handelt.

9. Verfahren nach einem der Ansprüche 6 bis 8, **dadurch gekennzeichnet, dass** der Fettstoffgehalt zwischen 10 und 99 Gew.-%, bezogen auf das Gewicht der Zusammensetzung, liegt.

10. Verfahren nach einem der vorhergehenden Ansprüche, **dadurch gekennzeichnet, dass** man auf die Keratinfasern eine durch unmittelbares Mischen der ersten und zweiten Zusammensetzung zum Zeitpunkt der Anwendung erhaltene Zusammensetzung aufbringt.

11. Verfahren nach einem der Ansprüche 1 bis 9, **dadurch gekennzeichnet, dass** man die beiden Zusammensetzungen nacheinander und ohne Zwischenspülung auf die Keratinfasern aufbringt.

12. Zusammensetzung, die einen Wassergehalt von weniger als 10 Gew.-% aufweist und eine oder mehrere Aminosiliciumverbindungen der folgenden Formel (I): 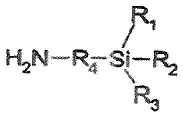 worin:
R₁, R₂ und R₃ gleich oder verschieden sind und für:
- einen linearen oder verzweigten C₁-C₂₀-Alkoxyrest, dessen Alkylteil gegebenenfalls durch ein oder mehrere Sauerstoffatome unterbrochen ist,
- einen linearen oder verzweigten C₂-C₂₀- und vorzugsweise C₂-C₄-Alkenyloxyrest stehen,
R₄ für einen zweiwertigen Rest der Formel: 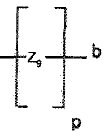
worin:
■ Z₉ für einen linearen C₁-C₂₀-Alkylenrest steht,
■ p gleich 1 ist,
■ a für die Bindung an das Siliciumatom steht,
■ b für die Bindung an das Stickstoffatom der Aminogruppe steht,
steht,
und einen oder mehrere Fettstoffe umfasst.

13. Vorrichtung mit mehreren Kompartimenten, die in mindestens einem Kompartiment eine Zusammensetzung gemäß Anspruch 12 und in mindestens einem anderen Kompartiment eine Zusammensetzung, die ein oder mehrere Oxidationsmittel umfasst, umfasst.
